# EUROPEAN PATENT SPECIFICATION

(11) **EP 4 027 858 B1**
(45) Date of publication and mention of the grant of the patent: **25.10.2023**
(21) Application number: 20767857.4
(22) Date of filing: 11.09.2020
(51) Int. Cl.: A61B 1/00, A61B 1/06, A61B 1/07, A61B 1/12

(54) **A DEVICE FOR VISUALIZATION OF INTERNAL TISSUE OF A PATIENT**
VORRICHTUNG ZUR VISUALISIERUNG VON INNEREM GEWEBE EINES PATIENTEN
DISPOSITIF DE VISUALISATION D'UN TISSU INTERNE D'UN PATIENT

(30) Priority: 13.09.2019 EP 19197320
(43) Date of publication of application: 20.07.2022
(73) Proprietor: LiNA Medical International Operations AG, 6039 Root D4 (CH)
(72) Inventor: MADSEN, Mads, 2670 Greve (DK); BJØRN-RASMUSSEN, Peter, 2600 Glostrup (DK); POULSEN, Henrik, Bisgaard, 3550 Slangerup (DK)
(74) Representative: Inspicos P/S
(86) International application number: PCT/EP2020/075502
(87) International publication number: WO 2021/048378

(56) References cited:
- CN-A- 109 171 617
- JP-B1- S4 828 517
- US-A- 5 329 936
- US-A1- 2006 171 693
- US-A1- 2014 088 371
- US-A1- 2015 282 701
- US-A1- 2018 063 387
- US-A1- 2019 175 007
- US-A1- 2019 261 845

## Description

### INTRODUCTION

The present invention relates to a device for use in endoscopic procedures, inter alia in hysteroscopy, and particularly to a device for visualization of internal tissue of a patient, e.g. in the uterus, cervix, urethra, or the bladder. The device comprises a hand-held control unit, an elongated member, an illumination structure, and an image capturing tip. The control unit is dimensioned to be held by a user's hand, and the elongated member has a proximal end connected to the hand-held control unit and a distal end connected to the image capturing tip.

### BACKGROUND

Gynaecologists and urologists use various scopes in standard examination procedures, both office-based procedures and in hospital procedures where a scope is inserted e.g. into the uterus for inspecting the lining.

For the practitioner, the field of diagnostic imaging, for example hysteroscopy, has allowed for the viewing of objects, internal mechanisms, and the like with minimal disruption.

The scopes on the market normally use integrated lamps e.g. based on Light emitting diodes (LED) for illuminating the field of view. While an LED may provide an energy efficient illumination and a small size compared e.g. with other light sources, the downside of using an LED is that excessive heating is generated, and the need for efficient cooling may complicate the device and make the device heavier and impair ergonomic conditions. Document US 2018/063387 A1 discloses a device for visualization of internal tissue as set out in the preamble of claim 1.

### DESCRIPTION OF THE INVENTION

It is an object of the invention to provide an improved device for visualization of internal tissue e.g. relative to manoeuvrability, light conditions, heat generation, and ergonomic conditions as set out in claim 1.

Accordingly, the invention, provides a device wherein the illumination structure comprises at least a first optical fibre and a second optical fibre. Each of the first and second optical fibres extends along the elongated member and forms a releasing fibre end at the image capturing tip and a receiving fibre end at the control unit. The receiving fibre end is in communication with an LED in a light interface in the control unit and light from the LED can thereby enter the fibre.

The elongated member is typically slim and space through the elongated member for tools, cables, or fluid etc. is limited. Since the LED is in the control unit as opposed to being at the distal end of the elongated member, space in the distal end of the elongated member can be saved and heating problems are moved away from the patient. Since the light from the LED is distributed via at least two separate optical fibres, a relatively high cross sectional area of the total optical fibre distribution can be obtained with a small cross sectional area of each fibre. This enables very flexible optical fibres, and a small cross section of the elongated member.

Due to the relatively high cross section area of the combined first and second optical fibres, a larger percentage of the light generated in the LED can be transmitted. That reduces the necessary lumen from the LED, and a less powerful LED can be used even with a slim elongated member, e.g. suitable for insertion though cervix etc. It thereby further reduces the heat generation and potentially reduces cost. Further, due to the two releasing fibre ends, as opposed to one single releasing fibre end, the light can be emitted with less generation of shadows.

The visualization device may particularly form a hysteroscope for inspection of uterus, or it may form a cystoscope for visual inspection of the bladder or a urinary tract.

Herein, the term hand-held means that it is capable of being held and manipulated by the hand of the surgeon. The hand-held control unit may particularly be shaped as a handle for manipulation of the device with one hand. The entire devise may particularly be a portable, handheld, and completely independent device having all functions necessary for performing the intended visualization embedded in one single unit. The control unit may particularly be independently powered by a battery, and it may be fitted with different parts such as a monitor, a fluid flushing system, and other parts which are suitable for the procedure. In that way, the device may form a complete, independent, visualization device, e.g. suitable for single use.

The elongated member may be shaped to match the specific purpose. For a hysteroscopic procedure, the elongated member may e.g. be less than 5 mm. in a cross section. Typically, it may have a circular cross section with a diameter of e.g. 4,0-4,8 mm, and a length of approximately 240 mm or more. The elongated member could be made of steel, e.g. with a coating of a low frictional polymer material. The elongated member may form a working channel extending from an inlet at the hand-held control unit to a distal tool opening at or in the image capturing tip. The working channel may allow insertion of a surgical tool through the elongated member, e.g. a forceps or similar tools for taking samples or removing tissue. In one embodiment, the invention provides the combination of a device for visualisation and forceps which are insertable through the working channel.

The purpose of the illumination structure is to provide light suitable for capturing images internally in the patient. The Kelvin Range: 3500-6000K is suitable e.g. for hysteroscopy. Particularly, a Kelvin Range of 5000-6000K may be suitable, and particularly when measured at the releasing fibre end.

The image capturing tip may be constituted by a separate tip-element containing an image capturing structure e.g. in the form of a camera, e.g. a CCD (Charged Coupled Device) and a camera lens. The separate tip-element may include a flow structure for release of fluid from the image capturing tip and for entering fluid into the elongated member via the image capturing tip. Again, in combination with fluid flow, e.g. for distending the bladder or for flushing blood and tissue away from the image capturing tip, optical fibres may allow illumination in a robust manner without the risk of short circuiting electrical connections at the image capturing tip and without risking too high temperatures caused by the heating of an LED at the image capturing tip.

The image capturing tip may communicate video signals with a monitor, e.g. a monitor attached to or forming part of the control unit.

The illumination structure comprises an LED which is housed in the control unit. In that way, heating generated by the LED is at a distance from the patient, and outside the body of the patient. The LED can be powered, e.g. by a battery in the control unit, without having to draw cables through the elongated member. That saves space in the narrow, elongated, member.

The first optical fibre and a second optical fibre may be glass fibres or polymer fibres etc. Particularly they may be thin fibres having a cross section with a size below 1 mm, or below 0,8 mm or below 0,6 mm. They may have a circular cross section, and they may be coated with a reflective material in the lengthwise direction between the ends.

The optical fibres extend along and inside the elongated member.

The releasing fibre ends are the ends of the fibres located at the image capturing tip where the light is released internally in the patient. The releasing fibre ends could be fixed to the image capturing tip or it could be embedded in the image capturing tip.

The opposite free end, herein referred to as a receiving fibre end, is arranged in communication with the LED, i.e. such that light from the LED can enter the fibres.

The elongated member may be rotationally attached to the control unit to allow reorientation of the image capturing tip. In that case, the optical fibres may form an over-length defined as a length exceeding the distance between the LED and the image capturing tip. By means of the over-length, the elongated member can be rotated while the optical fibres are twisted. The over-length may e.g. form a loop of optical fibre in the control unit. The loop may have a length allowing at least 360 degrees rotation of the elongated member while twisting the over-length about the elongated member.

In an alternative embodiment, the LED is attached to the rotatable elongated member and rotates therewith. In that embodiment, electrical supply cables to the LED may have an over-length allowing them to twist during rotation.

In yet another alternative embodiment, the light interface between the LED and the optical fibres is rotational such that the receiving fibre end at the control unit can rotate relative to the LED.

The releasing fibre ends may be encapsulated or potted in a tip defining an optic for distribution of light. In one embodiment, however, the releasing fibre ends are exposed at the image capturing tip such that the fibre ends themselves create the light distribution.

The image capturing tip may comprise a CCD, e.g. formed with an integrated lens. The CCD is configured to communicate video signals with a monitor. The CCD and the releasing fibre ends could be arranged in a row on a front face of the image capturing tip. By definition herein, the row direction is the direction defined by a line extending between the CCD and the releasing fibre ends with the releasing fibre ends on opposite sides of the CCD. The CCD may particularly be located between the releasing fibre ends. The elongated member may, as mentioned above, define a working channel extending from an entrance in the proximal end to an exit in the distal end. The exit in the distal end may be shifted relative to the row in a direction perpendicular to the row direction such that the exit is below or above the array of the CCD and the releasing fibre ends. The optical fibres may particularly be plastic fibres with a diameter of less than 1 mm, such as less than 0,6 mm, such as 0,5 mm. Plastic fibres of this size may particularly be an advantage if the optical fibres extend along an outer surface of the elongated member since plastic may be more durable than glass fibres and less problematic e.g. if they break or become detached from the elongated member in the body.

The light interface may comprise a PCB having an upper surface with a connector to which the LED is attached. The PCB may further include an area of metal exposed on a surface of the PCB, e.g. on the upper surface or on an opposite lower surface. The upper surface may be a planar surface, and the LEDs could be connected to this area to allow conduction of heat away from the LED. By the term *"exposed"* is herein meant that the PCB has an area of metal which is not covered by components. I.e. the LED is not attached directly over the exposed metal area but connected to this metal area by some sort of connection, e.g. connected to a metal area being adjacent to the exposed metal area. The exposed metal area may release thermal energy and the PCB may therefore function as a heat sink for the LED.

The PCB may particularly have a relatively large thickness layer of the copper layer, e.g. 70 µm Cu, or above 70µm Cu. The high thickness may increase heat conductivity and further facilitate cooling of the LED.

The light interface may comprise an interface element through which the first optical fibre and the second optical fibre extend. In one embodiment, the interface element comprises one or more through holes extending from an inner surface to an outer surface of the interface element and the optical fibres extend through the holes. The interface element may comprise a first through hole and a second through hole, the first optical fibre being fixed in the first through hole and the second optical fibre being fixed in the second through hole. Alternatively, both the first and second optical fibre may extend through the same through hole.

The interface element may include cooling ribs or surface irregularities, including dots or projections increasing the surface area of the interface element.

In another embodiment, the interface element comprises a slit from the inner to the outer surface and extending from an edge of the interface element. The slit allows the interface element to be attached about the fibres without access to the free ends of the fibres. That may be useful if the receiving ends are already attached to the LED.

In one embodiment, the interface element is used for the attachment of the optical fibres to the LED. In this embodiment, the optical fibres are attached, e.g. adhesively to the interface element, and the interface element is attached to the PCB, e.g. by screws, rivets, or adhesively.

The interface element may particularly be fixed to the PCB with the inner surface in direct contact with the PCB, e.g. in direct contact with the area of metal which functions as a heat sink for the LED.

The LED may project upwards from the upper surface of the PCB, and the inner surface of the interface element may comprise a recess and the LED may be located in that recess such that the inner surface can follow the upper surface of the PCB, particularly in direct contact with the surface of the PCB.

The interface element could be made from a polymer material such as PC/ABS or such as Cycoloy C2800. Alternatively, the interface element could be made of metal or alloys of metal, e.g. aluminium.

The receiving fibre end of at least one of the first and the second optical fibres may be in direct contact with a light emitting surface of the LED. In that embodiment, the optical fibre(s) being in direct contact with the LED may conduct heat away from the LED and thereby reduce the temperature of the LED. In this embodiment, heat resistance of the optical fibre is essential, and it may be an advantage to make the fibre from glass or similar high temperature resistant material.

The receiving fibre end of at least one of the first and the second optical fibres may be elevated above the light emitting surface of the LED. In that embodiment, the optical fibre(s) being elevated above the LED may be less influenced by the heat of the LED and less temperature resistance of the fibre is required. In this embodiment, the fibre could be made e.g. from less temperature resistant polymer materials.

At least one of the PCB and the interface element may be in direct contact with a housing of the control unit. The PCB and/or the interface element may e.g. be connected adhesively, by screws, by rivets, or by other means to the housing Particularly, the aforementioned metal area forming a heat sink for the LED may be in direct contact with the housing.

The elongated member may form a straight portion extending along a straight axis and a curved portion forming a curvature away from the straight axis. The curved portion could be between the image capturing tip and the straight portion. In this embodiment, the use of two optical fibres as opposed to one optical fibre enables use of thinner optical fibres for the same optical throughput. Accordingly, the bending of the optical fibres may be easier when comparing with devices having only one, thicker, optical fibre.

The elongated member comprises an inner tube and an outer tube. The inner tube is rotational in the outer tube and it may comprise a rigid inner section axially coextending a flexible outer section of the outer tube. The rigid inner section may have a curvature which forms the curvature of the curved portion by deflection of the flexible outer section. In this embodiment, the first and second optical fibres may particularly be located outside the inner tube and particularly with no direct contact to the surface of the inner tube. This may enable the rotation of the inner tube in the outer tube without wearing the optical fibres.

In the present invention, the outer tube is fixed to a flange of a body of the image capturing tip, and the inner tube is rotationally suspended to a bearing structure of the body of the image capturing tip. In the present invention, the first and second optical fibres may extend between the flange and the bearing structure.

Herein, the term *"power density"* refers to the ratio between the power consumed by the LED and the total area of the two receiving ends, i.e. twice the area of one fibre cross section. The LED may be configured to consume an effect in the range of 200-1500 mWatt and both the first and the second optical fibre have a diameter between 0,4 and 0,6 mm. A diameter of each fibre being 0,5 mm. provides a cross sectional area in the size of approx. 0,2 mm², and a power density in the range of: 200/0,2-1500/0,2. I.e. 1000-7500 mW/mm².

The device may comprise a flow structure for flow of liquid used for flushing the image capturing structure and/or for extending the uterus. The flow structure may comprise a flow conduit extending close to, and in heat communication with, the interface element, the LED, or the PCB. The flow conduit may e.g. be a rubber or silicone hose. By the location of the flow conduit in heat communication with the LED, the liquid may cool the LED and increase the lifetime of the LED. Simultaneously, the LED can heat the liquid which may be advantageous to provide a more comfortable treatment. In one embodiment, the flow conduit is in direct contact with at least one of the interface element, the LED, the PCB, and/or an optional battery.

The device may comprise a battery for powering the image capturing tip and the LED, and a controller for processing data from the image capturing tip. It may also comprise communication means, e.g. comprising wireless transmission means, for transmitting captured images to external systems. The controller and battery may be housed e.g. in the control unit. If the battery is housed in the control unit, the distance between the LED and the battery can be kept low, which reduces the energy loss in cables. Further, heating from the LED may be absorbed partly by the battery.

There is also disclosed a method of transferring light in a device that does not form part of the present invention. The method comprises transferring a first light intensity from an LED in the control unit via a first fibre to a delivering fibre end of the first fibre at the image capturing tip, transferring a second light intensity from the LED in the control unit via a second fibre to a delivering fibre end of the second fibre at the image capturing tip. The first and second light intensities may be equal or different from each other.

### LIST OF DRAWINGS

Figs. 1 and 2 illustrate an internal tissue visualization device;
Figs. 3a, 3b, and 3c illustrate different embodiments of the distal end of the elongated member;
Figs. 4a and 4b illustrate internal components in the control unit;
Figs. 5-7 illustrate details of the light interface and PCB; and
Fig. 8 illustrates an alternative location of the releasing fibre ends.

### DETAILED DESCRIPTION

It should be understood that the detailed description and specific examples, while indicating embodiments of the invention, are given by way of illustration only, since various changes and modifications within the scope of the invention as defined in the appended set of claims will become apparent to those skilled in the art from this detailed description.

In the following, embodiments of the invention will be described in further details with reference to the drawing in which:

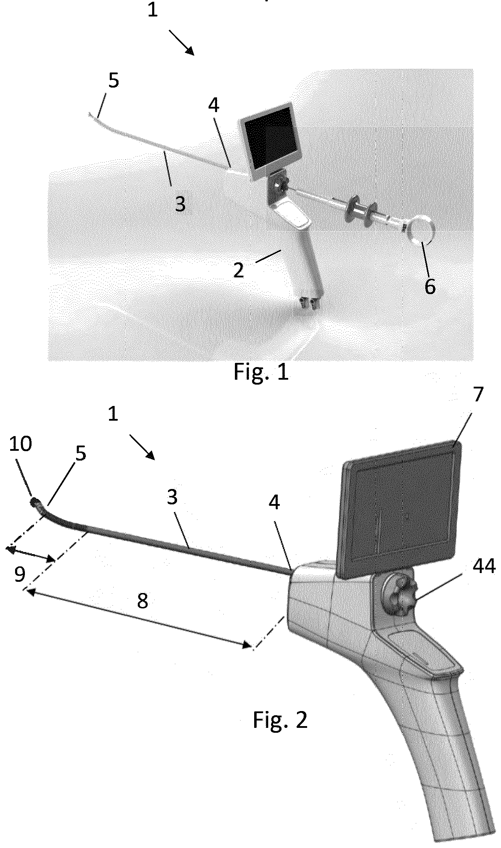

Figs. 1 and 2 illustrate an internal tissue visualization device according to the invention.

In Fig. 1, a tool inserted in the working channel;
The tissue visualization device 1 comprises an image capturing structure configured to capture pictures of tissue.

The device 1 comprises a hand-held control unit 2 and an elongated member 3 connected to the control unit 2. The elongated member extends from a proximal end 4 to a distal end 5. The distal end forms curved portion with an angle of 20 degrees to the straight axis. The curved portion 9 is between the straight portion 8 and the image capturing tip 10. The image capturing tip comprises a CCD which is configured to provide electronic video signals for the monitor 7.

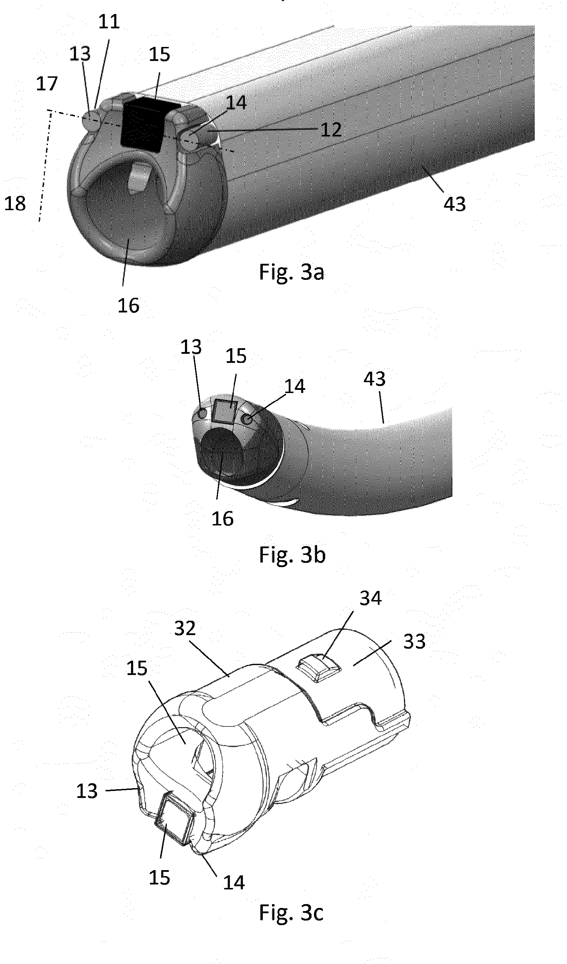

Fig. 3 illustrates the distal end of the elongated member 3 and the image capturing tip 10. The device comprises a first optical fibre 11, and a second optical fibre 12. Each fibre extends from a receiving fibre end inside the control unit to a releasing fibre end, 13, 14 at the image capturing tip 10. The CCD 15 is placed between the releasing fibre ends 13, 14.

The device forms a working channel having an exit 16. The releasing ends of the optical fibres and the CCD are arranged along a straight line illustrated by the dotted line 17, and the exit is shifted downwards relative to the row in a direction perpendicular to the row direction and illustrated by the dotted line 18.

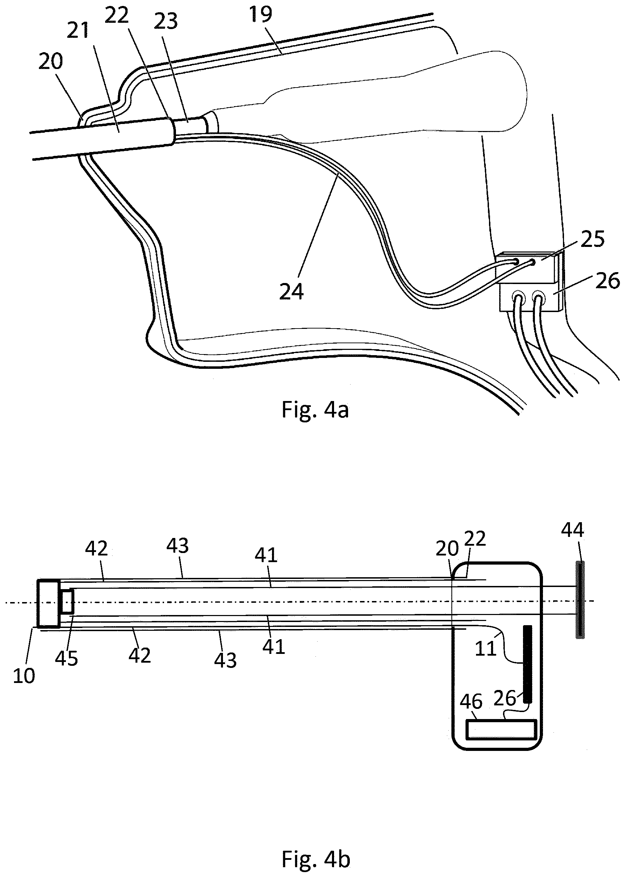

Fig. 3c illustrates the body 32 of the image capturing tip without the elongated member and in an embodiment where the elongated member is made from an inner and an outer tube, as described relative to Fig. 4b. In this embodiment, the outer surface 33 of the image capturing tip body 32 is fixed to the outer tube and the inner tube is rotationally suspended in the body 32. The body 32 forms a fixed engagement outer tube and is rotationally locked relative to the outer tube via the projection 34. The outer tube and at least a proximal part of the body 32 is covered by a hose 43 (not illustrated in Fig. 3C), e.g. of a shrink wrap material, and the optical fibres are located between the hose and the outer surface of the outer tube.

Figs. 4a and 4b illustrate internal components in the control unit. The control unit forms an encapsulating shell 19 forming an opening at 20. The elongated member 3 extends through the opening and is suspended in the inner space inside the control unit. The elongated member 3 is covered with the sheath 21 of shrink wrap terminating at 22 and leaving a portion 23 of the elongated member uncovered. The sheath extends on both sides of the opening and covers also the optical fibres 11, 12 and thereby protects the fibres from the edges of the shell 19.

As c illustrated, the fibres form a slack loop 24 in the housing and allow the elongated member to be rotated while the fibres are wound around the elongated member.

The optical fibres end at the light interface 25 which is attached to the PCB 26. The PCB 26 carries the LED. The PCB is connected to a battery 42 located in the control unit.

Fig. 4b illustrates an embodiment comprising an inner tube 41 and an outer tube 42. The inner tube 41 is rotational in the outer tube 42 and it comprises a rigid inner section axially coextending a flexible outer section of the outer tube. The rigid inner section may have a curvature which forms the curvature of the curved portion by deflection of the flexible outer section. This is not illustrated in Fig. 4b, but it can be seen in Figs. 1 and 2. In this embodiment, the first and second optical fibres may particularly be located outside the inner tube and particularly with no direct contact to the surface of the inner tube. This may enable the rotation of the inner tube in the outer tube without wearing the optical fibres. In the illustrated embodiment, the optical fibres are located outside the outer tube 42 and fixed thereon by a hose 43 attached about the outer tube. The inner tube can be rotated by use of the handle 44, also shown in Fig. 2, and the inner tube may e.g. be suspended rotationally in the bearing structure 45 provided in the image capturing tip 10, and the outer tube 42 is fixed to the image capturing tip and thereby prevents rotation of the image capturing tip.

Fig. 4b only illustrates one of the two fibres 11, 12, but both fibres may be located outside the outer tube.

The LED is powered by the battery 46.

The LED is powered by the battery 46. The battery may be directly connected to one of the PCB 26, the light interface 25 or the optic fibres, and thereby further function as a heat zinc. Further, the length of the electrical cable between the battery and the LED may be much shorter than the length of the optic fibres. Short electrical connectors reduce electrical interference.

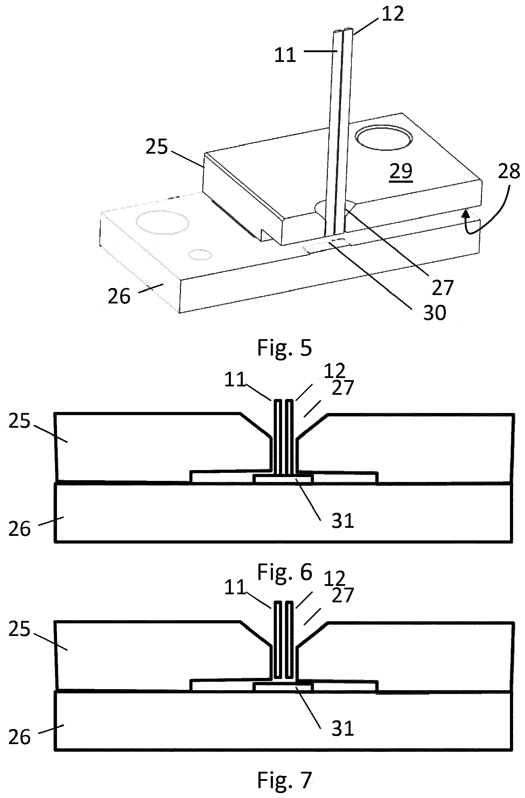

The light interface and LED are illustrated in further details in Figs. 5-7. The light interface 25 comprises a single through hole 27 in which the receiving ends of the optical fibres are placed. The first optical fibre and the second optical fibre extend through the interface element from an inner surface 28 to an outer surface 29 of the interface element.

The receiving ends are preferably glued into place in the light interface, and extend to the upper, light emitting, surface 30 of the LED 31.

Fig. 6 illustrates an embodiment where the receiving end is directly against the upper surface 30. In this embodiment, the hot upper surface will be in good heat conducting contact with the fibre end, and the fibre end will therefore contribute in distributing the thermal energy from the LED to the light interface. The optical fibres may e.g. be glass fibres having a high melting point compared e.g. with common plastic fibres. The advantage of having direct contact is further that the light from the LED is emitted better and with a low rate of loss. Accordingly, the LED may have less power consumption and thus heat emission for the same intensity of the light.

Fig. 7 illustrates an embodiment where the receiving end is lifted away from the upper surface 30. In this embodiment, the hot upper surface will be in poor heat conducting contact with the fibre end, and the fibre end will therefore not contribute in distributing the thermal energy from the LED to the light interface. The optical fibres may e.g. be plastic fibres having a low melting point compared e.g. with glass fibres. The distance between the receiving fibre end and the LED reduces the emission rate from the LED to the fibre but also reduces the heat emission from the LED to the fibre.

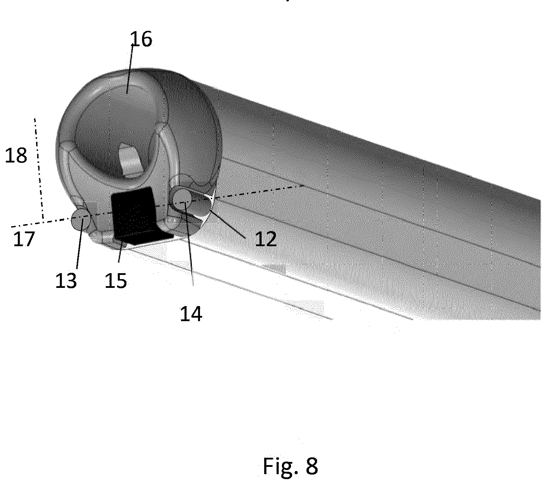

Fig. 8 illustrates an embodiment, where the releasing fibre ends are located below the exit 16 of the working channel.

## Claims

1. A device (1) for visualization of internal tissue, the device comprising a hand-held control unit (2), an elongated member (3), an illumination structure, and an image capturing tip (10), the control unit being dimensioned to be held by a user's hand, the elongated member having a proximal end (4) connected to the hand-held control unit and a distal end (5) connected to the image capturing tip, wherein the illumination structure comprises an LED housed in the control unit, and at least a first optical fibre (11) and a second optical fibre (12), each of the first optical fibre and the second optical fibre extending along the elongated member and forming a releasing fibre end (13, 14) at the image capturing tip and a receiving fibre end at the control unit, the receiving fibre end being in communication with the LED in a light interface in the control unit, **characterized in that** the elongated member comprises an inner tube (41) and an outer tube (42), the outer tube being fixed to a flange of a body of the image capturing tip, the inner tube being rotationally suspended to a bearing structure of the body of the image capturing tip, and the first and second optical fibres extending between the flange and the bearing structure.

2. The device according to claim 1, wherein the optical fibres form an over-length exceeding the distance between the LED and the image capturing tip.

3. The device according to claim 2, wherein the over-length forms a slack-loop in the control unit.

4. The device according to any of the preceding claims, wherein the image capturing tip comprises a CCD, and wherein the CCD and the releasing fibre ends are arranged in a row extending in a row direction with the releasing fibre ends on opposite sides of the CCD.

5. The device according to claim 4, wherein the elongated member forms a working channel extending from an entrance in the proximal end to an exit in the distal end, and wherein the exit is shifted relative to the row in a direction perpendicular to the row direction.

6. The device according to any of the preceding claims, wherein the light interface comprises a PCB defining an area of metal exposed on an essentially planar surface of the PCB and the LED being connected to the area to lead heat away from the LED.

7. The device according to any of the preceding claims, wherein the light interface comprises an interface element, the first optical fibre and the second optical fibre extending through the interface element from an inner surface to an outer surface of the interface element.

8. The device according to claim 6 and 7, wherein the interface element is fixed to the PCB with the inner surface in direct contact with the PCB.

9. The device according to claim 8, wherein the interface element is attached in direct contact with the area of metal.

10. The device according to any of claims 6-9, wherein the interface element comprises a first through hole and a second through hole, the first optical fibre being fixed in the first through hole and the second optical fibre being fixed in the second through hole.

11. The device according to any of claims 6-9, wherein the interface element comprises one single through hole, and wherein the first optical fibre and the second optical fibre are fixed in the single through hole.

12. The device according to any of claims 7-11, wherein the inner surface of the interface element comprises a recess and the LED extends into the recess.

13. The device according to any of the preceding claims, wherein the elongated member and the first and second optical fibres are covered by a polymer hose.

14. The device according to any of the preceding claims, wherein the elongated member forms a straight portion (8) extending along a straight axis and a curved portion (9) forming a curvature away from the straight axis, the curved portion being between the image capturing tip and the straight portion, the inner tube being rotational in the outer tube and comprising a rigid inner section axially coextending a flexible outer section of the outer tube, the rigid inner section having a curvature which forms the curvature of the curved portion by deflection of the flexible outer section, and wherein the first and second optical fibres are located outside the inner tube and with no direct contact to the surface of the inner tube.

## Patentansprüche

1. Vorrichtung (1) zur Visualisierung von innerem Gewebe, wobei die Vorrichtung eine handgehaltene Steuereinheit (2), ein längliches Element (3), eine Beleuchtungsstruktur und eine Bildaufnahmespitze (10) umfasst, wobei die Steuereinheit so dimensioniert ist, dass sie von der Hand eines Benutzers gehalten werden kann, wobei das längliche Element ein proximales Ende (4) aufweist, das mit der handgehaltenen Steuereinheit verbunden ist, und ein distales Ende (5), das mit der Bildaufnahmespitze verbunden ist, wobei die Beleuchtungsstruktur eine in der Steuereinheit untergebrachte LED und mindestens eine erste optische Faser (11) und eine zweite optische Faser (12) umfasst, wobei sich sowohl die erste optische Faser als auch die zweite optische Faser entlang des länglichen Elements erstrecken und ein freigebendes Faserende (13, 14) an der Bildaufnahmespitze und ein aufnehmendes Faserende an der Steuereinheit bilden, wobei das aufnehmende Faserende mit der LED in einer Lichtschnittstelle in der Steuereinheit in Verbindung steht,
**dadurch gekennzeichnet, dass** das längliche Element eine innere Röhre (41) und eine äußere Röhre (42) umfasst, wobei die äußere Röhre an einem Flansch eines Körpers der Bildaufnahmespitze befestigt ist, die innere Röhre drehbar an einer Tragestruktur des Körpers der Bildaufnahmespitze aufgehängt ist, und die erste und zweite optische Faser sich zwischen dem Flansch und der Tragestruktur erstrecken.

2. Vorrichtung nach Anspruch 1, wobei die optischen Fasern eine Überlänge bilden, die den Abstand zwischen der LED und der Bildaufnahmespitze überschreitet.

3. Vorrichtung nach Anspruch 2, wobei die Überlänge einen Slack-Loop in der Steuereinheit bildet.

4. Vorrichtung nach einem der vorstehenden Ansprüche, wobei die Bildaufnahmespitze ein CCD umfasst, und wobei das CCD und die freigebenden Faserenden in einer Reihe angeordnet sind, die sich in einer Reihenrichtung erstreckt, wobei die freigebenden Faserenden auf gegenüberliegenden Seiten des CCD liegen.

5. Vorrichtung nach Anspruch 4, wobei das längliche Element einen Arbeitskanal bildet, der sich von einem Eingang im proximalen Ende zu einem Ausgang im distalen Ende erstreckt, und wobei der Ausgang relativ zur Reihe in einer Richtung senkrecht zur Reihenrichtung verschoben ist.

6. Vorrichtung nach einem der vorstehenden Ansprüche, wobei die Lichtschnittstelle eine Leiterplatte umfasst, die einen Bereich aus Metall definiert, der auf einer im Wesentlichen ebenen Fläche der Leiterplatte frei liegt, und die LED mit dem Bereich verbunden ist, um Wärme von der LED abzuleiten.

7. Vorrichtung nach einem der vorstehenden Ansprüche, wobei die Lichtschnittstelle ein Schnittstellenelement umfasst, die erste optische Faser und die zweite optische Faser sich durch das Schnittstellenelement von einer inneren Fläche zu einer äußeren Fläche des Schnittstellenelements erstrecken.

8. Vorrichtung nach Anspruch 6 und 7, wobei das Schnittstellenelement an der Leiterplatte mit der inneren Fläche in direktem Kontakt mit der Leiterplatte befestigt ist.

9. Vorrichtung nach Anspruch 8, wobei das Schnittstellenelement in direktem Kontakt mit dem Metallbereich befestigt ist.

10. Vorrichtung nach einem der Ansprüche 6 - 9, wobei das Schnittstellenelement ein erstes Durchgangsloch und ein zweites Durchgangsloch umfasst, wobei die erste optische Faser im ersten Durchgangsloch befestigt ist und die zweite optische Faser im zweiten Durchgangsloch befestigt ist.

11. Vorrichtung nach einem der Ansprüche 6 - 9, wobei das Schnittstellenelement ein einziges Durchgangsloch umfasst, und wobei die erste optische Faser und die zweite optische Faser in dem einzigen Durchgangsloch befestigt sind.

12. Vorrichtung nach einem der Ansprüche 7 - 11, wobei die innere Fläche des Schnittstellenelements eine Aussparung umfasst und die LED sich in diese Aussparung erstreckt.

13. Vorrichtung nach einem der vorstehenden Ansprüche, wobei das längliche Element und die erste und zweite optische Faser von einem Polymerschlauch abgedeckt sind.

14. Vorrichtung nach einem der vorstehenden Ansprüche, wobei das längliche Element einen geraden Abschnitt (8) bildet, der sich entlang einer geraden Achse erstreckt, und einen gekrümmten Abschnitt (9), der eine Krümmung weg von der geraden Achse bildet, wobei der gekrümmte Abschnitt zwischen der Bilderfassungsspitze und dem geraden Abschnitt liegt, wobei die innere Röhre in der äußeren Röhre drehbar ist und einen starren inneren Abschnitt umfasst, der sich axial mit einem flexiblen äußeren Abschnitt der äußeren Röhren erstreckt, wobei der starre innere Abschnitt eine Krümmung aufweist, welche die Krümmung des gekrümmten Abschnitts durch Biegung des flexiblen äußeren Abschnitts bildet, und wobei die erste und zweite optische Faser außerhalb der inneren Röhre und ohne direkten Kontakt mit der Oberfläche der inneren Röhre angeordnet sind.

## Revendications

1. Dispositif (1) de visualisation d'un tissu interne, le dispositif comprenant une unité de commande manuelle (2), un membre allongé (3), une structure d'éclairage et un embout de capture d'image (10), l'unité de commande étant dimensionnée pour être tenue par la main d'un utilisateur, le membre allongé ayant une extrémité proximale (4) reliée à l'unité de commande manuelle et une extrémité distale (5) reliée à l'embout de capture d'image, dans lequel la structure d'éclairage comprend une LED logée dans l'unité de commande, et au moins une première fibre optique (11) et une seconde fibre optique (12), chacune de la première fibre optique et de la seconde fibre optique s'étendant le long du membre allongé et formant une extrémité de fibre de desserrage (13, 14) sur l'embout de capture d'image et une extrémité de fibre de réception à l'unité de commande, l'extrémité de fibre de réception étant en communication avec la LED dans une interface de lumière dans l'unité de commande,
**caractérisé en ce que** le membre allongé comprend un tube intérieur (41) et un tube extérieur (42), le tube extérieur étant fixé sur une bride d'un corps de l'embout de capture d'image, le tube intérieur étant suspendu en rotation à une structure porteuse du corps de l'embout de capture d'image, et les première et seconde fibres optiques s'étendant entre la bride et la structure porteuse.

2. Dispositif selon la revendication 1, dans lequel les fibres optiques forment une sur-longueur excédant la distance entre la LED et l'embout de capture d'image.

3. Dispositif selon la revendication 2, dans lequel la sur-longueur forme une boucle lâche dans l'unité de commande.

4. Dispositif selon l'une quelconque des revendications précédentes, dans lequel l'embout de capture d'image comprend un CCD, et dans lequel le CCD et les extrémités de fibre de desserrage sont agencés dans une rangée s'étendant dans une direction de rangée avec les extrémités de fibre de desserrage sur des côtés opposés du CCD.

5. Dispositif selon la revendication 4, dans lequel le membre allongé forme un canal de travail s'étendant d'une entrée dans l'extrémité proximale à une sortie dans l'extrémité distale, et dans lequel la sortie est décalée par rapport à la rangée dans une direction perpendiculaire à la direction de rangée.

6. Dispositif selon l'une quelconque des revendications précédentes, dans lequel l'interface de lumière comprend un PCB définissant une zone de métal exposé sur une surface essentiellement planaire du PCB et la LED étant connectée à la zone pour diriger la chaleur à distance de la LED.

7. Dispositif selon l'une quelconque des revendications précédentes, dans lequel l'interface de lumière comprend un élément d'interface, la première fibre optique et la seconde fibre optique s'étendant à travers l'élément d'interface d'une surface intérieure à une surface extérieure de l'élément d'interface.

8. Dispositif selon la revendication 6 et 7, dans lequel l'élément d'interface est fixé au PCB avec la surface intérieure en contact direct avec le PCB.

9. Dispositif selon la revendication 8, dans lequel l'élément d'interface est fixé en contact direct avec la zone de métal.

10. Dispositif selon l'une quelconque des revendications 6-9, dans lequel l'élément d'interface comprend un premier trou traversant et un second trou traversant, la première fibre optique étant fixée dans le premier trou traversant et la seconde fibre optique étant fixée dans le second trou traversant.

11. Dispositif selon l'une quelconque des revendications 6-9, dans lequel l'élément d'interface comprend un unique trou traversant et dans lequel la première fibre optique et la seconde fibre optique sont fixées dans l'unique trou traversant.

12. Dispositif selon l'une quelconque des revendications 7-11, dans lequel la surface intérieure de l'élément d'interface comprend un creux et la LED s'étend dans le creux.

13. Dispositif selon l'une quelconque des revendications précédentes, dans lequel le membre allongé et les première et seconde fibres optiques sont couverts par un tuyau en polymère.

14. Dispositif selon l'une quelconque des revendications précédentes, dans lequel le membre allongé forme une partie droite (8) s'étendant le long d'un axe droit et une partie incurvée (9) formant une courbe à distance de l'axe droit, la partie incurvée étant entre l'embout de capture d'image et la partie droite, le tube intérieur étant rotatif dans le tube extérieur et comprenant une section intérieure rigide co-étendant axialement une section extérieure flexible du tube extérieur, la section intérieure rigide ayant une courbe qui forme la courbure de la partie incurvée par fléchissement de la section extérieure flexible, et dans lequel les première et seconde fibres optiques sont situées hors du tube intérieur et sans contact direct avec la surface du tube intérieur.
